(19) Europäisches Patentamt
European Patent Office
Office européen des brevets

(11) EP 4 674 652 A1

(12) EUROPEAN PATENT APPLICATION

(43) Date of publication:
07.01.2026 Bulletin 2026/02

(21) Application number: 24315326.9

(22) Date of filing: 04.07.2024

(51) International Patent Classification (IPC):
B60H 1/00 (2006.01)    G01N 15/06 (2024.01)
G01N 15/00 (2024.01)

(52) Cooperative Patent Classification (CPC):
G01N 15/06; B60H 1/008; B60H 1/00828;
G01N 15/075; G01N 2015/0046

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: MANN+HUMMEL Ventures Pte. Ltd.
Singapore 139234 (SG)

(72) Inventors:
• Woitoll, Ina
71636 Ludwigsburg (DE)

• Kunze, Stefan
71636 Ludwigsburg (DE)
• Zenner, Julien
53000 Laval (FR)
• Michel, Christiane
71636 Ludwigsburg (DE)

(74) Representative: Mann + Hummel Intellectual
Property
Mann + Hummel
International GmbH & Co. KG
Schwieberdinger Straße 126
71636 Ludwigsburg (DE)

(54) **DEVICE AND METHOD FOR ESTIMATING AND UTILIZING ULTRA-FINE PARTICLE CONCENTRATION**

(57) Aspects concern a method for estimating and utilizing ultra-fine particle concentration, the method (500) comprising: receiving (501) data indicative of, a measured ultra-fine particle concentration and a measured nitrogen oxide concentration, the measured ultra-fine particle concentration and the measured nitrogen oxide concentration correlated to each location of a plurality of locations and corresponding time of a plurality of times; establishing (502) a correlation between ultra-fine particle concentration and nitrogen oxide concentration from the measured ultra-fine particle concentrations and the measured nitrogen oxide concentrations; estimating (503), from a nitrogen oxide concentration measured at a location where the ultra-fine particle concentration is to be estimated, the ultra-fine particle concentration according to the correlation; and comparing (504) the estimated ultra-fine particle concentration with at least one ultra-fine particle concentration threshold to determine whether the estimated ultra-fine particle concentration is higher or lower than the at least one ultra-fine particle concentration threshold.

FIG. 5

**Description**

**TECHNICAL FIELD**

Various aspects of this disclosure relate to devices and methods for estimating and utilizing an ultra-fine particle concentration.

**BACKGROUND**

The following discussion of the background art is intended to facilitate an understanding of the present disclosure only. It should be appreciated that the discussion is not an acknowledgement or admission that any of the material referred to was published, known or is part of the common general knowledge of the person skilled in the art in any jurisdiction as of the priority date of the disclosure.

For a vehicle, it may be desirable to have information about ultra-fine particles (UFP) in its surrounding air, e.g. for controlling a cabin air system.

While air quality stations exist that may provide information about pollution on the street level, those may not cover every location, at least not for all types of pollution. For example, a certain local measurement station may only provide information about a current local pollution status regarding defined parameters, such as PM10, PM2.5, or gas concentration, which thus may in particular not cover the concentration of ultra-fine particles (UFP). Measuring UFP may be difficult and may require a relatively high amount of technical effort.

Moreover, there is at present no checking or feedback mechanism to determine if a measurement station, which may comprise one or more sensors, is working correctly, and if the cabin air system is activated at the moment when UFP concentration is deemed to be relatively high. A defined parameter, such as PM 2.5, may also not have the same sources as UFPs and may not have any correlation with UFPs.

Accordingly, efficient and accurate approaches for estimating UFP concentration and/or utilizing the estimated UFP concentration are desirable.

**SUMMARY**

The present disclosure seeks to provide a technical solution for estimating ultra-fine particles (UFP) in an environment, for example, a location of a vehicle. The estimated UFP may be compared against one or more defined thresholds. The compared results may be used for controlling a vehicle system, such as a cabin air system of the vehicle.

Various embodiments concern a method for estimating and utilizing ultra-fine particle concentration, the method comprising: receiving data indicative of, a measured ultra-fine particle concentration and a measured nitrogen oxide concentration, the measured ultra-fine particle concentration and the measured nitrogen oxide concentration correlated to each location of a plurality of locations and corresponding time of a plurality of times; establishing a correlation between ultra-fine particle concentration and nitrogen oxide concentration from the measured ultra-fine particle concentrations and the measured nitrogen oxide concentrations; estimating, from a nitrogen oxide concentration measured at a location where the ultra-fine particle concentration is to be estimated, the ultra-fine particle concentration according to the correlation; and comparing, the estimated ultra-fine particle concentration with at least one ultra-fine particle concentration threshold to determine whether the estimated ultra-fine particle concentration is higher or lower than the at least one ultra-fine particle concentration threshold.

To facilitate an end-consumer oriented approach, one or more UFP concentration thresholds may be defined. In some embodiments, two or three thresholds may be implemented.

In some embodiments, the at least one ultra-fine particle concentration threshold comprises a first threshold and a second threshold, and wherein the first threshold is associated with switching on a vehicle system, and the second threshold is associated with switching off the vehicle system, and optionally wherein the at least one ultra-fine particle concentration between the first threshold and the second threshold is associated with a standby mode of the vehicle system.

In some embodiments, the second threshold is less than the first threshold, and wherein in a positive determination that the estimated ultra-fine particle concentration is greater or equal to the first threshold, a first control signal to switch on the vehicle system is generated; or in a positive determination that the estimated ultra-fine particle concentration is less than the second threshold, a second control signal to switch off the vehicle system is generated, and optionally wherein the vehicle system is a cabin air system having one or more filters, and the method comprises controlling the cabin air system by sending the first control signal to switch on the cabin air system or the second control signal to switch off the vehicle system.

In some embodiments, the first control signal comprises an intensity signal to increase or decrease a filtration strength in response to that the estimated ultra-fine particle concentration indicates that an ultra-fine particle concentration in air surrounding the vehicle system has increased and/or decreasing filtration strength in response to that the estimated ultra-fine particle concentration indicates that the ultra-fine particle concentration in air surrounding the vehicle system has decreased.

In some embodiments, estimating the ultra-fine particle concentration further comprises depending on the location where the ultra-fine particle concentration is to be estimated, a time parameter, and information about a weather condition at the location where the ultra-fine particle concentration is to be estimated.

In some embodiments, the nitrogen concentration is a nitrogen dioxide concentration, and/or a concentration of a mixture of nitrogen monoxide and nitrogen dioxide.

In some embodiments, the nitrogen concentration is a nitrogen dioxide concentration, and/or a concentration of a mixture of two or more NOx, where x is a positive integer. In some embodiments, the nitrogen concentration may also include nitrogen monoxide concentration alone.

In some embodiments, the method comprise receiving, by a server, a request for an estimate of the ultra-fine particle concentration, wherein the request indicates the nitrogen oxide concentration, and providing the estimated ultra-fine particle concentration in response to the request.

In some embodiments, the method further comprises measuring, by a vehicle sensor, the nitrogen oxide concentration at the location where the ultra-fine particle concentration is to be estimated, or by requesting, from a database, the nitrogen oxide concentration at the location where the ultra-fine particle concentration is to be estimated. In some embodiments, the vehicle sensor may include one or more NOx sensors. The one or more NOx sensors may be configured to obtain a measurement of a concentration of a mixture of two or more NOx. As a non-limiting example, the measurement may include a concentration of a mixture of NO and $NO_2$.

In some embodiments, the method further comprises measuring, by a vehicle sensor, the nitrogen oxide concentration at the location where the ultra-fine particle concentration is to be estimated.

In some embodiments, the method further comprises requesting the nitrogen oxide concentration at the location where the ultra-fine particle concentration is to be estimated from a database.

In some embodiments, the method further comprises controlling a vehicle system using the estimated ultra-fine particle concentration. In some embodiments, the vehicle system may be a cabin air system.

In some embodiments, controlling the cabin air system comprises increasing a filtration strength in response to that the estimated ultra-fine particle concentration indicates that an ultra-fine particle concentration in air surrounding the vehicle system has increased and/or decreasing filtration strength in response to that the estimated ultra-fine particle concentration indicates that the ultra-fine particle concentration in air surrounding the vehicle system has decreased. The increasing of the filter strength may mean increasing an air airflow through a filter element. The increased airflow may be achieved via increasing a blower speed of a suction device, and/or opening one or more airflow control flaps.

According to one embodiment, controlling the cabin air system comprises increasing a filter strength. The increasing of the filter strength may mean increasing an air airflow through a filter element. The increased airflow may be achieved via increasing a blower speed of a suction device, and/or opening one or more airflow control flaps in response to that the estimated ultra-fine particle concentration indicates that an ultra-fine particle concentration in air surrounding the vehicle system (e.g. air surrounding the vehicle or, in case that there is no vehicle sensor in the vehicle, a NOx sensor external to the vehicle) has increased and/or decreasing filter strength in response to that the estimated ultra-fine particle concentration indicates that the ultra-fine particle concentration in air surrounding the vehicle system has decreased. (So, for example, cabin air system air performance may be increased in case of higher pollution (in particular UFP concentrations) or, in case of lower pollution, its performance may be decreased to lower energy consumption in case of less pollution; it is also possible to keep a fresh air intake closed when driving through an area with high pollution).

In some embodiments, estimating the ultra-fine particle concentration further comprises depending on the location where the ultra-fine particle concentration is to be estimated, a time parameter, and information about a weather at the location where the ultra-fine particle concentration is to be estimated.

According to one embodiment, the method comprises requesting the NOx concentration at the location where the ultra-fine particle concentration is to be estimated from a database. For example, electronic data of a map of UFP concentrations which may be calculated from NOx concentrations, or the NOx concentrations data, may be obtained and/or updated continuously and from the map, the UFP concentration, or an estimation of the UFP concentration based on the stored NOx concentration may be obtained. So, a vehicle may not need to have a NOx sensor and GPS coordinates may be sufficient to send from the vehicle to a server that gathers the NOx concentration elsewhere. The gathered NOx concentration may then be used by the server or the vehicle to derive the UFP concentration.

According to another aspect of the present disclosure there is provided a data processing system configured to perform the method as described.

According to another aspect of the present disclosure there is provided a cabin air system controller for a cabin air system of a vehicle, the cabin air system controller comprising at least one processor configured to obtain a measurement of a nitrogen oxide concentration at a location of the vehicle; send a request, from the controller, to a server, for an estimate of an ultra-fine particle concentration based on the nitrogen oxide concentration; receive, by the controller, an estimated ultra-fine particle concentration in response to the request, the estimated ultra-fine particle concentration determined according to any one of the method as described; send a request, from the controller, to a comparator module, to compare the estimated ultra-fine particle concentration with the at least one ultra-fine particle concentration threshold to determine whether the estimated ultra-fine particle concentration is higher or lower than the at least one ultra-fine particle concentration threshold; receive, by the controller, a control signal in response to the request; and control a filtration system of the cabin air system based on the control signal.

According to one embodiment, the cabin air system controller comprises at least one processor configured to obtain a measurement of a NOx concentration at a location of the vehicle, request an estimate of an ultra-fine particle concentration based on the NOx concentration (e.g. from a server computer (e.g. a cloud), i.e. a remote service, but also an onboard processor: correlation functions (lookup data) may also be pre-loaded and optionally kept updated to the vehicle, e.g., according to a pre-defined route, so that processing may, for example, be at the edge and not at the cloud. This has the benefit that real-time GPS location data need not be shared outside the vehicle for that purpose, increasing privacy. On the other hand, real-time information gives more fine-tuned concentration data and better control of the cabin air system.), receive an estimated ultra-fine particle concentration in response to the request, and control a cabin air system using the estimated ultra-fine particle concentration.

In some embodiments, the at least one processor is configured to receive nitrogen oxide concentration from a remote data source.

In some embodiments, the remote data source is at least one of a measuring point or from an air pollution map.

In some embodiments, the measurement of a nitrogen oxide concentration at the location of the vehicle is compared with or appended to the nitrogen oxide concentration received from the remote data source.

According to another aspect of the present disclosure there is provided a vehicle comprising a cabin air system, the cabin air system comprising a cabin air system controller as described, and wherein optionally, the vehicle further comprises a nitrogen oxide concentration sensor to obtain the measurement of a nitrogen oxide concentration, and/or a mixture of two or more NOx concentration, at the location of the vehicle.

In some embodiments, the at least one processor is configured to receive nitrogen oxide concentration from a remote data source, and optionally, wherein the remote data source is at least one of a measuring point from an air pollution map.

In some embodiments, the measurement of a nitrogen oxide concentration at the location of the vehicle is compared with or appended to the nitrogen oxide concentration received from the remote data source.

According to another aspect of the present disclosure there is provided a computer program element comprising program instructions, which, when executed by one or more processors, cause the one or more processors to perform the method as described.

According to another aspect of the present disclosure there is provided a computer-readable medium comprising program instructions, which, when executed by one or more processors, cause the one or more processors to perform the method as described.

It should be noted that embodiments described in context of the method is analogously valid for the devices (vehicle, cabin air system controller, data processing system) and vice versa.

## BRIEF DESCRIPTION OF THE DRAWINGS

The disclosure will be better understood, with reference to the detailed description, when considered in conjunction with the non-limiting examples and the accompanying drawings, in which:

- FIG. 1 shows a vehicle with a cabin air system.
- FIG. 2 shows a flow diagram illustrating an estimation of UFP (ultra-fine particle) concentration based on nitrogen oxide.
- FIG. 3 and FIG. 4 show diagrams illustrating the correlation between NOx concentration and UFP concentration near a busy street.
- FIG. 5 shows a flow diagram illustrating a method for estimating an ultra-fine particle concentration.

## DETAILED DESCRIPTION

The following detailed description refers to the accompanying drawings that show, by way of illustration, specific details and embodiments in which the disclosure may be practiced. These embodiments are described in sufficient detail to enable those skilled in the art to practice the disclosure. Other embodiments may be utilized and structural, and logical changes may be made without departing from the scope of the disclosure. The various embodiments are not necessarily mutually exclusive, as some embodiments can be combined with one or more other embodiments to form new embodiments.

Embodiments described in the context of one of the devices or methods are analogously valid for the other devices or methods. Similarly, embodiments described in the context of a device are analogously valid for a vehicle or a method, and vice-versa.

Features that are described in the context of an embodiment may correspondingly be applicable to the same or similar features in the other embodiments. Features that are described in the context of an embodiment may correspondingly be applicable to the other embodiments, even if not explicitly described in these other embodiments. Furthermore, additions and/or combinations and/or alternatives as described for a feature in the context of an embodiment may correspondingly be applicable to the same or similar feature in the other embodiments.

In the context of various embodiments, the articles "a", "an" and "the" as used with regard to a feature or element include a reference to one or more of the features or elements.

As used herein, the term "and/or" includes any and all combinations of one or more of the associated listed items.

As used herein, the term "vehicle" includes a motor-powered vehicle, for example a vehicle having 2, 3, 4, or more wheels. Examples of the vehicle include a passenger car, a truck, a bus, a lorry, an aircraft, or a rail vehicle, for example, a locomotive. In some embodiments, the vehicle may be an electric vehicle or a hybrid (internal combustion engine and electrical) vehicle. In some embodiments, the vehicle may be an internal combustion engine (ICE) vehicle (i.e., non-hybrid). The term vehicle may further include autonomous vehicles.

As used herein, the term "processor(s)", as used herein, includes one or more electrical circuits capable of processing data, i.e. processing circuits. A processor may include analog circuits or components, digital circuits or components, or hybrid circuits or components. Any other kind of implementation of the respective functions which will be described in more detail below may also be understood as a "circuit" in accordance with an alternative embodiment. A digital circuit may be understood as any kind of a logic implementing entity, which may be special purpose circuitry or a processor executing software stored in a memory, or a firmware.

As used herein, the term "module" refers to, or forms part of, or include an Application Specific Integrated Circuit (ASIC); an electronic circuit; a combinational logic circuit; a field programmable gate array (FPGA); a processor (shared, dedicated, or group) that executes code; other suitable hardware components that provide the described functionality; or a combination of some or all of the above, such as in a system-on-chip. The term module may include memory (shared, dedicated, or group) that stores code executed by the processor.

As used herein, the term "data" may be understood to include information in any suitable analog or digital form, for example, provided as a file, a portion of a file, a set of files, a signal or stream, a portion of a signal or stream, a set of signals or streams, and the like. The term data, however, is not limited to the aforementioned examples and may take some forms and represent any information as understood in the art.

As used herein, the term "obtain", refers to the processor which actively obtains the inputs, or passively receives inputs from one or more sensors or data source. The term obtain may also refer to a processor, which receives or obtains inputs from a communication interface, e.g. a user interface. The processor may also receive or obtain the inputs via a memory, a register, and/or an analog-to-digital port.

As used herein, the term "ultrafine particle(s)" (UFPs) refers to particulate matter of nanoscale size, typically less than 0.1 micro-meters ($\mu$m) or 100 nano-meters (nm) in diameter. The term "ultrafine particle concentration" may be understood as "any data indicative of concentration". In some embodiments, concentration may refer to the quantity of ultrafine particles present in a given volume of air. It may be measured in terms of particles per cubic meter (particles/m$^3$) or mass concentration (e.g., micrograms per cubic meter, $\mu$g/m$^3$).

As used herein, the term "sensor" may include at least one hardware sensor, at least one software sensor, or a combination of the at least one hardware and software sensors. In some embodiments, data from the at least one sensor may be remotely assessed.

As used herein, the term "cabin air system" may include a ventilation system installed in an enclosed space, such as an aircraft, automobile, train, or building. A primary function of the cabin air system may be to treat and circulate air within the enclosed space, i.e. providing filtered air after removal of pollutants such as dust, allergens, odors, and potentially harmful gases for occupants. In some embodiments, the cabin air system may also include regulation functions such as temperature or humidity regulation. In some embodiments, the cabin air system may include air conditioning units, fans, filters, and ducting to distribute air throughout the cabin. In some embodiments, the cabin air system may include components such as the heating, ventilation, and/or air conditioning (HVAC) system, which draws in outside air, filters it, and then circulates it through the cabin to maintain a comfortable environment for passengers.

In the following, embodiments will be described in detail.

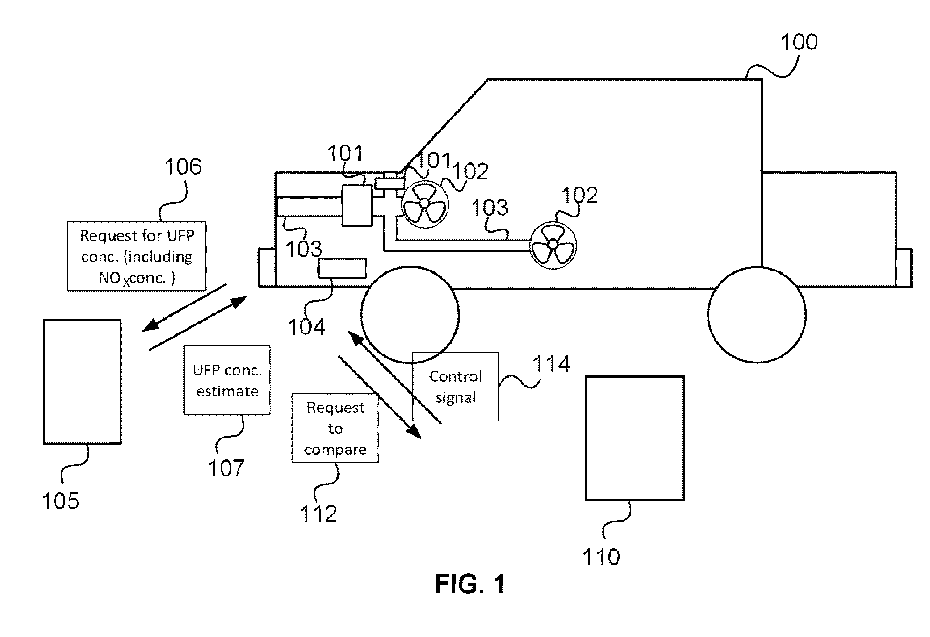

FIG. 1 shows a vehicle 100 with a cabin air system.

The cabin air system comprises one or more filters 101, fans 102, and ducts 103. In some embodiments, the filters 101, fans 102, and ducts 103 may be arranged in series to work together to provide and circulate air throughout the vehicle's cabin. The cabin air system is designed to remove pollutants, dust, and other harmful particles from the air while also regulating temperature and humidity levels. In particular, the cabin air system operates as a cabin air filtration system and may be regarded as such. Similarly, the cabin air system may operate and be regarded as an in-vehicle ambient air filtration system. The ambient air may be understood as air external to the vehicle. In some embodiments, the fans 102 may be configured to produce airflow containing ultra-fine particles through the filters 101, i.e. the fans 102 may operate as suction devices/blowers to increase or decrease filtration strength of the filtration system. In some embodiments, the increase and decrease in filtration strength correspond respectively to an increased and decreased airflow drawn through the filters 101 over a period of time.

The cabin air system comprises a controller 104 which controls its operation by various control actions, e.g. sets fan speeds and the degree to which air vents and air ducts are opened. The controller 104 may for example measure interior air quality and perform corresponding control actions. In some embodiments, the controller 104 may be an electronic controller.

According to various embodiments, a more optimized cabin air (filtration) system is provided in that it takes into consideration not only the air quality within the cabin, but also the external air quality, in particular, ultra-fine particle concentration, to reduce health risks arising from breathing in the ultra-fine particles (UFPs).

Air Quality data may be available from public measurement stations. Such air quality data may include measurements like PM10 and PM2.5 and also $NO/NO_2$ measurements etc. The controller 104 of a cabin air system may obtain such information from the public measurement stations. However, ultra-fine particle concentration may not be available by these measurement stations since, for example, a certain local measurement station may only provide information about the current local pollution status relating to the defined parameters such as PM10, PM2.5 and/or gas concentrations. In most measurement stations, no information may be obtainable for particles smaller than PM2.5 particles, which may not have the same sources as UFP and therefore do not sufficiently correlate with the UFP to give accurate information about the UFP concentration.

Moreover, it should be noted that due to their relatively smaller size and mass, gravimetric or optical measurement methods, which are common for relatively larger sizes of fine dust, are typically unsuitable for measuring UFP. Some standardized methods for detecting and measuring the number concentration of UFP in air include particle counting methods and size classification based on electrical mobility. The measurement of a particle number size distribution allows an accurate assignment of particle sizes and the computational determination of a UFP number concentration over arbitrary particle size intervals. Mobility particle size spectrometers have a measurement uncertainty of up to about 10 percent in the range of the particle number concentration and down to a few percent in the range of the determined particle diameter. Below 10 nanometers (nm), greater uncertainties occur with the current state of the art. Continuous operation of mobility particle size spectrometers in the context of air monitoring requires regular maintenance and calibration by trained personnel. In the meantime, low-cost mobile measuring instruments that estimate the particle number concentration on the basis of an ion current measurement are also increasingly being found, but this is associated with reduced accuracy compared to condensation particle counters. Beyond the aforementioned real-time measuring instruments, it is possible in principle, albeit with greater technical effort, to examine UFP chemically or to produce electron microscope images.

In view of the above, it may be difficult to measure UFP concentration, especially in a vehicle. Moreover, fewer measuring stations are typically available for UFP compared to PM2.5 and PM10.

Even in case the vehicle has a vehicle sensor for UFP concentration, it may be desirable to obtain information about UFP concentration from an additional source since otherwise, no (cross) check whether the sensor is working correctly is possible. So, an approach to estimate UFP concentration is desirable to ensure that the cabin air system (or one of its components such as a HEPA filter) is activated at the moment when UFP concentration is relatively high. In some embodiments, the UFP concentration may be compared against one or more defined thresholds.

Therefore, according to various embodiments, an estimation method, which may include an estimation algorithm, may be used to determine the local UFP concentration at a location where the vehicle 100 is located. The determination of the local UFP concentration may be achieved by one or more onboard controllers of the vehicle 100, for example, controller 104, or may be via a server 105. In some embodiments, the server 105 may be a remote server may be a cloud server. In some embodiments, the determination includes an estimation of the local UFP concentration.

According to some embodiments, the estimation method uses a correlation between the nitrogen oxide concentration (e.g. NOx, where x may be 1 and/or 2) and at least one of the following data: a geographical location, a time parameter, such as a time of day, a day of the week and a season. In some embodiments, the geographical location may include a country, a suburb, a city, an airport, a road, a roadside, a mountain region. In some embodiments, the time parameter may include a time stamp, i.e. a computer time corresponding to that the NOx data is obtained.

In an embodiment, the vehicle 100 may send a request 106, such as an electronic request query, for the determination of an UFP concentration to the server 105. The vehicle 100 may measure or determine, e.g. by querying or consulting a reference database, the NOx concentration at its location and include it into the request 106. Alternatively, the vehicle may only include its location into the request 106 and the server 105 determines the NOx concentration itself. In any case, the server 105 or server arrangement may determine the UFP concentration by estimating the UFPU concentration from the NOx concentration and the correlation between these two concentrations and sends the UFP concentration estimate 107 to the vehicle. In some embodiments, the server 105 may include a requestor processor to process the request 106, and a server processor to return the UFP concentration estimate 107.

In some embodiments, the location data may be obtained based on global positioning system (hereinafter referred to as GPS). By using GPS location data and nitrogen and NOx data, which may include concentration data of a mixture of NOx, such as NO and/or $NO_2$ concentration, e.g. from a vehicle sensor or from a remote data source, for example, an official source or an air pollution map provider, the estimation method algorithm can calculate the UFP concentration. Thus, a variable that is currently difficult to measure, the UFP concentration, can be determined by using a "virtual sensor". It is appreciable that the data obtained from the vehicle sensor may be compared with the data obtained from the remote data source. In some embodiments, the data obtained from the vehicle sensor may be augmented with the remote data, such that the accuracy or amount of information of both the local and/or remote data may be enhanced.

Further, from information about influence on UFP concentration of weather condition and/or ozone as well as the consideration of influencing factors like daytime, season and (as mentioned above) location (e.g. environment) may be used in the UFP concentration estimation to make a more accurate estimation of the UFP concentration. The weather condition may include one or more of the following: ambient air temperature, solar irradiation, wind speed, wind direction, humidity level, and/or precipitation.

Effects like solar radiation may have an impact on new particle formation (NPF) that may be relevant for the determination of UFP concentration. In the formation of new particles, primary UFPs are mainly generated by emissions such as traffic emissions, industry emissions, emissions arising from burning processes. Secondary UFPs are mainly generated by NPF. For a more precise prediction, a distinction between primary and secondary UFPs may be made.

The NOx concentration based on which UFP concentration is estimated may be obtained by using one or more sensors. In some embodiments, the sensor may be aNOx sensor for sensing a mixture of NO and $NO_2$. In some embodiments, such NOx sensor may be installed in the vehicle 100, i.e. as onboard sensors, or may be obtained from existing and/or historical data from freely accessible measuring points or from manufacturers of air pollution maps to determine the NOx concentration. For the latter approach, no additional installation space in the vehicle 100 would have to be used for one or more corresponding sensors. In terms of sustainability, this may also save an additional component in the vehicle 100 and thus conserve resources or space.

In case a UFP sensor is provided in the vehicle 100, the UFP sensor may in some embodiments be a small, inexpensive, and robust sensor for measuring UFP concentration.

In some embodiments, the estimation method may be used to monitor the correct functioning of the sensor and a malfunction of the sensor. The determination of malfunction of the sensor may indicate that a necessary replacement of the sensor is necessary. Such determination of malfunction may be reported to the end user (e.g. driver of the vehicle). This may provide a resource-saving option for the lifetime monitoring of a sensor in terms of sustainability.

In some embodiments, the estimated UFP concentration may be compared with one or more UFP concentration thresholds, via a request to compare 112, and return a control signal 114 based on the compared results. In some embodiments, in addition to the compared results, specific input(s) of one or more passengers of the vehicle 100 may be received by the controller 104, the specific input(s) may be used to determine the control signal 114. In some embodiments, the control signal 114 may include analog and/or digital signals. The control signal 114 may further include an enable filter device signal and a disable filter device signal. The control signal may be digital signals, such as binary signals. For example, the enable filter device signal may be a binary '1' signal, and the disable filter device signal may be a binary '0' signal. Alternatively, the enable filter device signal may be a binary '0' signal, and the disable filter device signal may be a binary '1' signal. In various embodiments, the enable filter device signal may further include a signal to pre-condition or stand by the filter device.

In some embodiments, the enable filter device signal and the disable filter device signal may be analog signals, and converted to the aforementioned binary signals via one or more analog to digital converters (ADC).

In some embodiments, the one or more UFP concentration thresholds may be pre-defined or dynamically defined, i.e. determined and/or changed during operation. The estimated UFP concentration may be compared against the one or more defined UFP concentration thresholds via a comparator module 110. In some embodiments, the comparator module 110 may be integrated with the server 105.

In some embodiments, three levels of UFP concentrations, based on two thresholds, may be defined. The three levels may correspond to a low UFP concentration level, an acceptable UFP concentration level, and a high UFP concentration level. A first threshold and a second threshold may be defined. The first threshold may correspond to a boundary UFP concentration between the acceptable UFP concentration level and the high UFP concentration level. A high UFP concentration level may indicate that a filtering mode of the cabin air system may be switched on to filter ambient air passing into the cabin. The second threshold may correspond to a threshold between the low UFP concentration level and the acceptable UFP concentration level. A low UFP concentration level will indicate that the filtering mode of the cabin air system may be switched off to stop filtering ambient air. In some embodiments, the acceptable UFP concentration level may be indicative that the filtering mode of the cabin air system may be switch to a standby mode in anticipation of the UFP concentration increasing towards the first threshold, i.e. the UFP concentration level trending towards high. It may be appreciable that the second threshold is less than the first threshold, and in a positive determination that the estimated ultra-fine particle concentration is greater or equal to the first threshold, a first control signal to switch on the vehicle system is generated; or in a positive determination that the estimated ultra-fine particle concentration is less than the second threshold, a second control signal to switch off the vehicle system is generated. In some embodiments, additional thresholds or levels may be defined as required. The corresponding control signals of such additional thresholds/levels may correspond to various intensities of the filtration strength.

In some embodiments, the first control signal may comprise an intensity signal to increase or decrease a filtration strength in response to the estimated ultra-fine particle concentration indicating that an ultra-fine particle concentration in air surrounding the vehicle system has increased and/or decreasing filtration strength in response to that the estimated ultra-fine particle concentration indicates that the ultra-fine particle concentration in air surrounding the vehicle system has decreased.

In some embodiments, the controller 104 may comprise one or more graphical user interface (GUI) to provide an indication of the air quality, based on the comparison result of the estimated UFP concentration and the defined thresholds. For example, any UFP concentration above the first threshold may correspond to a bad air quality, any UFP concentration below the second threshold may correspond to a good air quality, and any UFP concentration above the second threshold but below the first threshold will be deemed an acceptable air quality. In some embodiments, different colours may be displayed on the GUI to provide users with a further indicator of air quality. For example, a green indicator may correspond to good air quality, yellow may correspond to an acceptable air quality, red may correspond to a bad air quality, etc.

In some embodiments, further UFP concentration thresholds may be defined. For example, there may be a third UFP concentration threshold associated with a warning that the UFP concentration has increased beyond the filtration capability/capacity, and the vehicle 100 should move out of the location immediately.

In some embodiments, the switching on or off of the cabin air system may correspond with the switching on or off of one or more filter devices 101, such as HEPA filter devices.

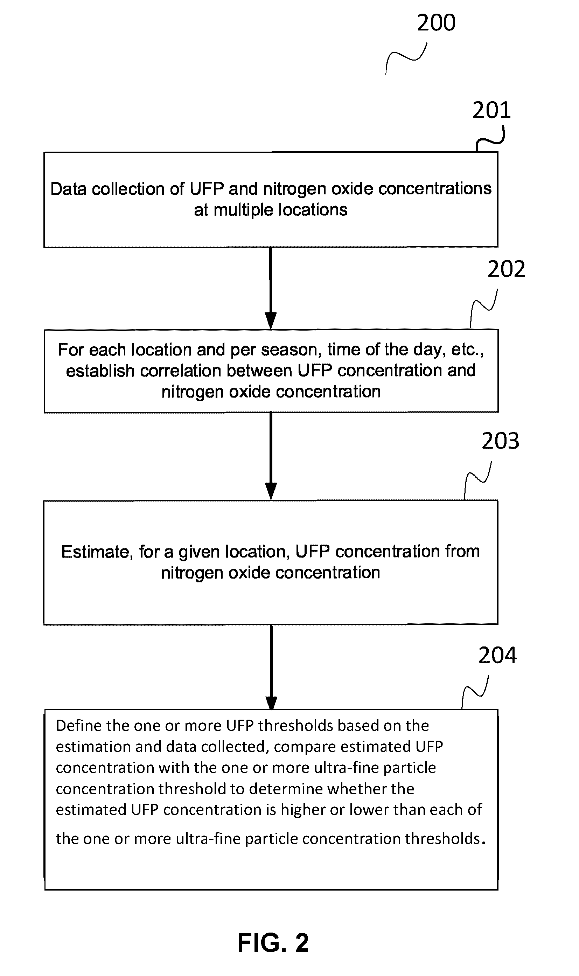

FIG. 2 shows a flow diagram 200 illustrating an estimation of UFP concentration based on NOx concentration, such as x = 1, i.e. NO concentration, and/or x= 2, $NO_2$ concentration.

In 201, one or more data measurements of UFP concentration and NOx concentration may be collected over a relatively long period of time (e.g. months or even years) from a plurality of different geographical locations, forming a historical dataset. It is appreciable that multiple data measurements may be collected at each of the plurality of different geographical locations.

In 202, a data processing unit may be arranged to obtain data measurements at each of the locations. The data processing unit may be a remote server or an onsite server. The data processing unit may be configured to perform one or more of the following functions:

- calculate statistical values such as average, mode, median, values of the data measurements over a predetermined period or time horizon. An example may be hourly averaged values for each day.
- determine or identify correlations of NOx concentration and UFP concentration based on one or more of the following criteria:

    ○ season- such as spring, summer, fall, winter
    ○ day of the week (or "weekday" and "weekend")
    ○ time of day (e.g. "night", "morning", "afternoon", "evening")
    ○ environment ("roadside", "mountain area", "urban area", "industrial area", "residential area")

In some embodiments, the correlation may be expressed mathematically in Equation (1) based on a linear dependency as follows:

$$\text{UFP\_concentration} = a * \text{nitrogen\_oxide\_concentration} + b \qquad (1)$$

where a and b are correlation constants. The data processing unit may be configured to determine the real numbers a and b based on each of the above criteria or for each combination thereof.

In 203, the data processing unit or another data processing unit, possibly in the vehicle 100 such as the controller 104, may use the determined correlations to determine UFP concentration based on nitrogen concentration NOx at a location. In some embodiments, the location may be different from the other locations at which the measurements were obtained. In some embodiments, the UFP concentration may be determined by using the determined values of a and b for the season, day of the week, time of the day and/or environment for which the UFP concentration should be determined (and the nitrogen concentration was measured).

In 204, the one or more UFP thresholds may be defined based on the Equation (1) and the statistical values of 202.

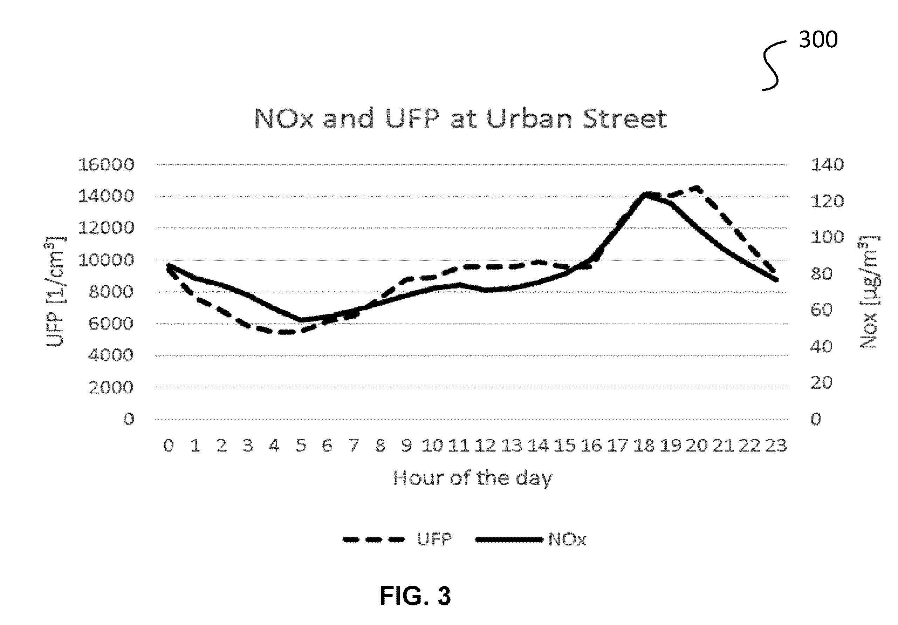

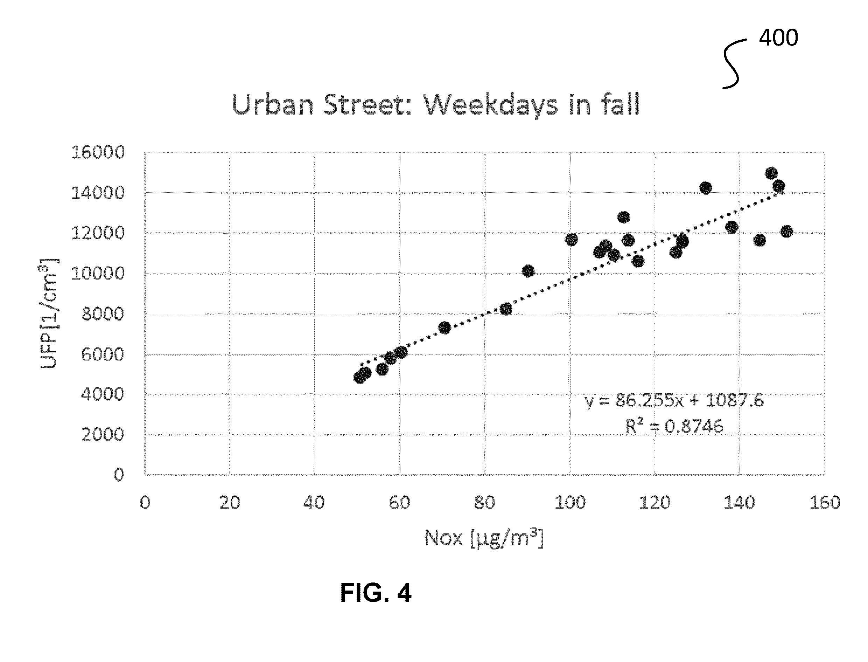

FIG. 3 and FIG. 4 show diagrams 300, 400 illustrating the correlation between NOx concentration and UFP concentration near an urban street, which may be relatively busy.

The diagram 300 of FIG. 3 includes hourly averages for the NOx and the UFP concentration of a weekday in a fall season.

The diagram 400 of FIG. 4 shows that UFP concentration may be well described or represented by a linear function from the NOx concentration. The constant a is determined to be 86.255 and the constant b is determined to be 1087.6. FIG. 4 illustrates the linear function obtained through a curve-fitting model, i.e. a regression model, with the $R^2$ value of 0.8746. As an alternative or additional embodiment, the historical dataset may be used to train a machine learning model. The machine learning model may be based on one or more of a supervised learning model, an unsupervised learning model, and/or a hybrid learning model. In some embodiments, the machine learning model may include one or more convolutional neural networks. It may be appreciable that the trained machine learning model may receive the NOx concentration as input and output the corresponding UFP concentration.

In summary, according to various embodiments, a method is provided as illustrated in 5.

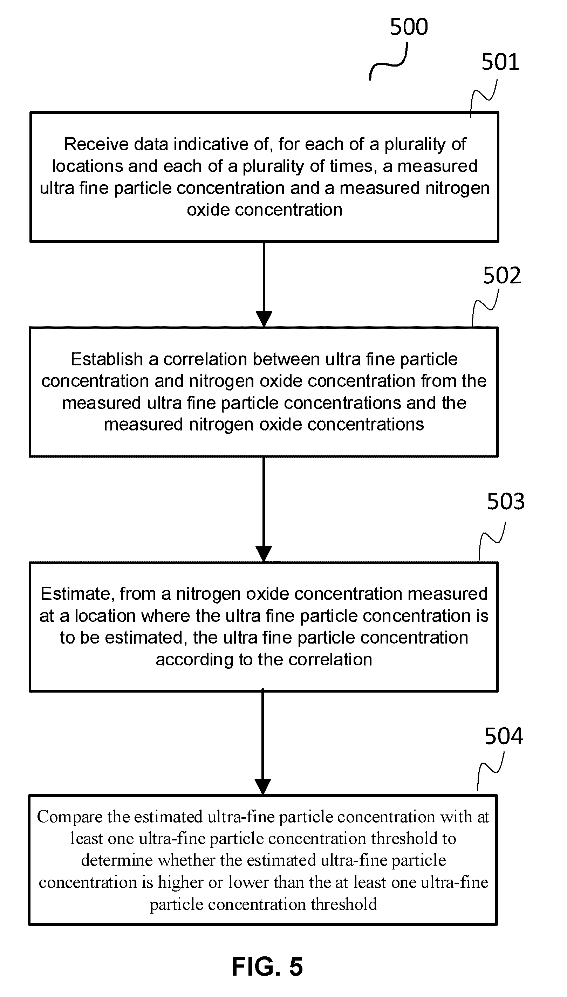

According to one embodiment, a method is provided as illustrated in FIG. 5.

FIG. 5 shows a flow diagram illustrating a method for estimating an ultra-fine particle concentration.

In 501, data is received indicative of, for each of a plurality of locations and each of a plurality of times, a measured ultra-fine particle concentration and a measured NOx concentration (this may include measuring the ultra-fine particle concentrations and the measured NOx concentrations and may, for example, be performed by a server computer). The measured ultra-fine particle concentration and the measured nitrogen oxide concentration may be correlated to/associated with at least one location of a plurality of locations, and a corresponding time of a plurality of times. As an example, the measured ultra-fine particle concentration and the measured nitrogen oxide concentration may be received from a road, in the afternoon 3pm (off-peak traffic hours) or in the morning 8am (peak traffic hours).

In 502 a correlation between ultra-fine particle concentration and NOx concentration is established from the measured ultra-fine particle concentrations and the measured NOx concentrations.

In 503, from aNOx concentration measured at a location where the ultra-fine particle concentration is to be estimated, the ultra-fine particle concentration is estimated according to the correlation. The estimation may include interpolation between correlations, or be based on corresponding formulas or models, like that of the parameters a and b in case of the linear model described in Equation (1) above, in case the UFP concentration should be determined at a location for which no correlation was established.

In 504, the estimated ultra-fine particle concentration may be compared with at least one ultra-fine particle concentration threshold to determine whether the estimated ultra-fine particle concentration is higher or lower than the at least one ultra-fine particle concentration threshold.

According to various embodiments, in other words, UFP concentration is estimated by exploiting the correlation of UFP concentration with NOx concentration. Thus, the estimation approach of FIG. 5 allows a precise estimation of the UFP concentration at a certain location by means of simple measurement technology, such as existing NOx sensors, without the need for complex measurement technology.

Besides being used, for example, for controlling a cabin air system, the estimated UFP concentration may also be displayed to a user via, for example, a user interface. So, instead of displaying air quality (AQ) on a dashboard or mobile application only based on measured values (either coming from external AQ measurement stations or from in-cabin sensor measurements the UFP estimation approach of FIG. 5 may be used to calculate an in-cabin AQ and display it without using additional sensors.

The estimated UFP concentration or a derivate thereof, such as an air quality index, which may be calculated on board or in a server, such as a remote cloud server, may also be made available at an API (Application Programming Interface) so that it can be accessed by other services. In some embodiments, the other services may include onboard or smartphone apps.

The methods described herein may be performed and the various processing or computation units and the devices and computing entities described herein may be implemented by one or more circuits. In an embodiment, a "circuit" may be understood as any kind of a logic implementing entity, which may be hardware, software, firmware, or any combination thereof. Thus, in an embodiment, a "circuit" may be a hard-wired logic circuit or a programmable logic circuit such as a programmable processor, e.g. a microprocessor. A "circuit" may also be software being implemented or executed by a processor, e.g. any kind of computer program, e.g. a computer program using a virtual machine code. Any other kind of implementation of the respective functions which are described herein may also be understood as a "circuit" in accordance with an alternative embodiment. A device (e.g. server) or arrangement (e.g. cabin system controller and server) which performs the method according to any of the embodiments may for example include one or more communication interfaces (to receive and send the various data and information e.g. position information, NOx concentration, UFP concentration estimate and control signals for the cabin air system) processing units and memories.

While the disclosure has been particularly shown and described with reference to specific embodiments, it should be understood by those skilled in the art that various changes in form and detail may be made therein without departing from the spirit and scope of the disclosure as defined by the appended claims. The scope of the disclosure is thus indicated by the appended claims and all changes which come within the meaning and range of equivalency of the claims are therefore intended to be embraced.

**Claims**

1. A method (500) for estimating and utilizing ultra-fine particle concentration, the method (500) comprising:

   receiving (501) data indicative of, a measured ultra-fine particle concentration and a measured nitrogen oxide concentration, the measured ultra-fine particle concentration and the measured nitrogen oxide concentration correlated to each location of a plurality of locations and corresponding time of a plurality of times;
   establishing (502) a correlation between ultra-fine particle concentration and nitrogen oxide concentration from the measured ultra-fine particle concentrations and the measured nitrogen oxide concentrations;
   estimating (503), from a nitrogen oxide concentration measured at a location where the ultra-fine particle concentration is to be estimated, the ultra-fine particle concentration according to the correlation; and
   comparing (504), the estimated ultra-fine particle concentration with at least one ultra-fine particle concentration threshold to determine whether the estimated ultra-fine particle concentration is higher or lower than the at least one ultra-fine particle concentration threshold.

2. The method (500) of claim 1, wherein the at least one ultra-fine particle concentration threshold comprises a first threshold and a second threshold, and wherein the first threshold is associated with switching on a vehicle system, and the second threshold is associated with switching off the vehicle system, and optionally wherein the at least one ultra-fine particle concentration between the first threshold and the second threshold is associated with a standby mode of the vehicle system.

3. The method (500) of claim 2, wherein the second threshold is less than the first threshold, and wherein in a positive determination that the estimated ultra-fine particle concentration is greater or equal to the first threshold, a first control signal to switch on the vehicle system is generated; or in a positive determination that the estimated ultra-fine particle concentration is less than the second threshold, a second control signal to switch off the vehicle system is generated, and optionally wherein the vehicle system is a cabin air system having one or more filters (101), and the method (500) comprises controlling the cabin air system by sending the first control signal to switch on the cabin air system or the second control signal to switch off the vehicle system.

4. The method (500) of claim 3, wherein the first control signal comprises an intensity signal to increase or decrease a filtration strength in response to that the estimated ultra-fine particle concentration indicates that an ultra-fine particle concentration in air surrounding the vehicle system has increased and/or decreasing filtration strength in response to that the estimated ultra-fine particle concentration indicates that the ultra-fine particle concentration in air surrounding the vehicle system has decreased.

5. The method (500) of any one of the preceding claims, wherein estimating the ultra-fine particle concentration further comprises depending on the location where the ultra-fine particle concentration is to be estimated, a time parameter, and information about a weather condition at the location where the ultra-fine particle concentration is to be estimated.

6. The method (500) of any one of the preceding claims, wherein the nitrogen concentration is a nitrogen dioxide concentration, and/or a concentration of a mixture of nitrogen monoxide and nitrogen dioxide.

7. The method (500) of any one of the preceding claims, comprising receiving, by a server (105), a request (106) for an estimate of the ultra-fine particle concentration (107), wherein the request (106) indicates the nitrogen oxide concentration, and providing the estimated ultra-fine particle concentration (107) in response to the request (106).

8. The method (500) of any one of the preceding claims, further comprises measuring, by a vehicle sensor, the nitrogen oxide concentration at the location where the ultra-fine particle concentration is to be estimated, or by requesting, from a database, the nitrogen oxide concentration at the location where the ultra-fine particle concentration is to be estimated.

9. A data processing system configured to perform the method of any one of claims 1 to 8.

10. A cabin air system controller (104) for a cabin air system of a vehicle (100), the cabin air system controller (104) comprising at least one processor (105) configured to

obtain a measurement of a nitrogen oxide concentration at a location of the vehicle;

sending a request (106), from the controller (104), to a server (105), for an estimate of an ultra-fine particle concentration based on the nitrogen oxide concentration;

receive, by the controller (104), an estimated ultra-fine particle concentration (107) in response to the request (106), the estimated ultra-fine particle concentration (107) determined according to any one of the method of claims 1 to 8;

sending a request (112), from the controller (104), to a comparator module (110), to compare the estimated ultra-fine particle concentration with the at least one ultra-fine particle concentration threshold to determine whether the estimated ultra-fine particle concentration is higher or lower than the at least one ultra-fine particle concentration threshold;

receive, by the controller (104), a control signal (114) in response to the request (112); and

control a filtration system of the cabin air system based on the control signal (114).

11. A vehicle (100) comprising a cabin air system (10, the cabin air system comprising a cabin air system controller (104) of claim 10, and wherein optionally, the vehicle (100) further comprises a nitrogen oxide concentration sensor to obtain the measurement of a nitrogen oxide concentration at the location of the vehicle (100).

12. The vehicle (100) of claim 11, wherein the at least one processor is configured to receive nitrogen oxide concentration from a remote data source, and optionally, wherein the remote data source is at least one of a measuring point from an air pollution map.

13. The vehicle (100) of claim 12, wherein the measurement of a nitrogen oxide concentration at the location of the vehicle (100) is compared with or appended to the nitrogen oxide concentration received from the remote data source.

14. A computer program element comprising program instructions, which, when executed by one or more processors, cause the one or more processors to perform the method of any one of claims 1 to 8.

15. A computer-readable medium comprising program instructions, which, when executed by one or more processors, cause the one or more processors to perform the method of any one of claims 1 to 8.

**FIG. 1**

EP 4 674 652 A1

200

201

Data collection of UFP and nitrogen oxide concentrations
at multiple locations

202

For each location and per season, time of the day, etc.,
establish correlation between UFP concentration and
nitrogen oxide concentration

203

Estimate, for a given location, UFP concentration from
nitrogen oxide concentration

204

Define the one or more UFP thresholds based on the
estimation and data collected, compare estimated UFP
concentration with the one or more ultra-fine particle
concentration threshold to determine whether the
estimated UFP concentration is higher or lower than each of

the one or more ultra-fine particle concentration thresholds.

FIG. 2

FIG. 3

400

Urban Street: Weekdays in fall

$y = 86.255x + 1087.6$
$R^2 = 0.8746$

Nox [µg/m³]

UFP [1/cm³]

16000
14000
12000
10000
8000
6000
4000
2000
0

0    20    40    60    80    100    120    140    160

**FIG. 4**

500

501

Receive data indicative of, for each of a plurality of locations and each of a plurality of times, a measured ultra fine particle concentration and a measured nitrogen oxide concentration

502

Establish a correlation between ultra fine particle concentration and nitrogen oxide concentration from the measured ultra fine particle concentrations and the measured nitrogen oxide concentrations

503

Estimate, from a nitrogen oxide concentration measured at a location where the ultra fine particle concentration is to be estimated, the ultra fine particle concentration according to the correlation

504

Compare the estimated ultra-fine particle concentration with at least one ultra-fine particle concentration threshold to determine whether the estimated ultra-fine particle concentration is higher or lower than the at least one ultra-fine particle concentration threshold

FIG. 5

Europäisches
Patentamt

European
Patent Office

Office européen
des brevets

# EUROPEAN SEARCH REPORT

**Application Number**

EP 24 31 5326

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MARVAL JESUS ET AL: "Ultrafine particles: A review about their health effects, presence, generation, and measurement in indoor environments", BUILDING AND ENVIRONMENT, PERGAMON PRESS, OXFORD, GB, vol. 216, 19 March 2022 (2022-03-19), XP087030137, ISSN: 0360-1323, DOI: 10.1016/J.BUILDENV.2022.108992 [retrieved on 2022-03-19] * section 3 "Outdoor vs. Indoor human exposure to the ultrafine fraction", second paragraph; section 6 "Conclusions", next to last paragraph; paragraph [0083] - paragraph [0096]; figure 7 * | 1-15 | INV. B60H1/00 G01N15/06 G01N15/00 |
| A | REGGENTE MATTEO ET AL: "Prediction of ultrafine particle number concentrations in urban environments by means of Gaussian process regression based on measurements of oxides of nitrogen", ENVIRONMENTAL MODELLING & SOFTWARE, ELSEVIER, AMSTERDAM, NL, vol. 61, 12 August 2014 (2014-08-12), pages 135-150, XP029047881, ISSN: 1364-8152, DOI: 10.1016/J.ENVSOFT.2014.07.012 * section 7 "Conclusions", first paragraph * | 1-15 | **TECHNICAL FIELDS SEARCHED (IPC)** B60H G01N |

-/--

The present search report has been drawn up for all claims

| Place of search | Date of completion of the search | Examiner |
|---|---|---|
| The Hague | 12 December 2024 | Eidmann, Gunnar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

**Application Number**

EP 24 31 5326

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CAN A ET AL: "Correlation analysis of noise and ultrafine particle counts in a street canyon", SCIENCE OF THE TOTAL ENVIRONMENT, ELSEVIER, AMSTERDAM, NL, vol. 409, no. 3, 1 January 2011 (2011-01-01), pages 564-572, XP027544768, ISSN: 0048-9697 [retrieved on 2010-12-02] * section 2 "Background", first paragraph * | 1-15 | |
| A | WO 2021/117946 A1 (DAEWOO LUCOMS CO LTD [KR]) 17 June 2021 (2021-06-17) * paragraph [0083] - paragraph [0096]; figure 7 * | 2-4 | |

TECHNICAL FIELDS
SEARCHED    (IPC)

The present search report has been drawn up for all claims

| Place of search | Date of completion of the search | Examiner |
|---|---|---|
| The Hague | 12 December 2024 | Eidmann, Gunnar |

EPO FORM 1503 03.82 (P04C01)

page 2 of 2

## ANNEX TO THE EUROPEAN SEARCH REPORT
## ON EUROPEAN PATENT APPLICATION NO.

EP 24 31 5326

This annex lists the patent family members relating to the patent documents cited in  the above-mentioned European search report.
The members are as contained in the European Patent Office EDP file on
The European Patent Office is in no way liable for these particulars which are merely given for the purpose of information.

12-12-2024

| Patent document cited in search report | Publication date | Patent family member(s) | Publication date |
|---|---|---|---|
| WO 2021117946 A1 | 17-06-2021 | KR 20210074859 A <br> WO 2021117946 A1 | 22-06-2021 <br> 17-06-2021 |

EPO FORM P0459

For more details about this annex : see Official Journal of the European Patent Office, No. 12/82